# EUROPEAN PATENT SPECIFICATION

(11) **EP 4 021 364 B1**
(45) Date of publication and mention of the grant of the patent: **20.09.2023**
(21) Application number: 19762118.8
(22) Date of filing: 28.08.2019
(51) Int. Cl.: A61F 13/15, A61F 13/49, A61F 13/514

(54) **ABSORBENT ARTICLE WITH BACKSHEET ARRANGEMENT**
SAUGFÄHIGER ARTIKEL MIT RÜCKFOLIENANORDNUNG
ARTICLE ABSORBANT AVEC AGENCEMENT DE FEUILLE ARRIÈRE

(43) Date of publication of application: 06.07.2022
(73) Proprietor: Essity Hygiene and Health Aktiebolag, 405 03 Göteborg (SE)
(72) Inventor: TERENTIC, Verica, 405 03 Göteborg (SE); MEYER NOREN, Rikard Vilhelm, 405 03 Göteborg (SE); ANDERSSON, Mikael, 405 03 Göteborg (SE); FREDERIX, Fred, 6598 BL Heijen (NL)
(74) Representative: Nederlandsch Octrooibureau
(86) International application number: PCT/EP2019/072942
(87) International publication number: WO 2021/037353

(56) References cited:
- WO-A1-96/09025
- US-A1- 2010 292 663

## Description

### TECHNICAL FIELD

The disclosure pertains to an absorbent article having first and second waist edges and first and second side edges, the absorbent article comprising a topsheet, a backsheet arrangement, an absorbent member being disposed between the topsheet and the backsheet arrangement, a first side flap extending from the first side edge in the lateral direction of the article and a second side flap extending from the second side edge in the lateral direction of the article, and first and second leg elastic members extending in the longitudinal direction of the absorbent article along first and second side edges of the backsheet arrangement. The first and second side flaps each has an inner end portion which is secured between the topsheet and the backsheet arrangement within first and second flap anchoring areas being arranged at one of both of the waist edges, each flap anchoring area having a lateral extension in the lateral direction of the absorbent article between an inner edge and an outer edge of the flap anchoring area and having a longitudinal extension in the longitudinal direction of the absorbent article between the first waist edge of the side flap and a corresponding leg edge of the side flap.

### BACKGROUND

Disposable absorbent articles, in particular disposable absorbent diapers which are worn in a pant-like fashion are commonly provided with elastic elements in selected areas, such as leg elastic arranged along leg edges of the absorbent article, and waist elastic arranged along the waist edge. Furthermore, the diapers may be provided with side flaps or belt elements comprising fastener elements for closing the absorbent garment when putting it on a user. Diapers with side flaps are for example disclosed in US2010/0292663 A1 and WO96/09025 A1.

Disposable articles having side flaps are usually produced in lengthwise production methods wherein the articles are continuously produced from continuous webs running in a machine direction which coincides with the length direction of the articles which are being produced. Continuous leg elastic elements are stretched and fed along with the traveling webs and are intermittently attached in their stretched state between a topsheet web and a backsheet web by means of adhesive or by ultrasonic welding or heat welding along the portions of the articles which are to form leg edges in the final absorbent article. Side flaps are attached at a front end and/or at a rear end of each precursor absorbent article in the moving web of precursor absorbent articles and are attached between the topsheet web and the backsheet web by means of adhesive or by ultrasonic welding or heat welding.

It is generally desired that the leg elastic elements are present in the final absorbent article only along the leg edges and not in front and rear body portions of the absorbent article close to the front and rear waist edges. For this reason, the leg elastic elements are bonded to the topsheet web and the backsheet web only where functional leg elastic is desired. In a final production step when individual absorbent articles are cut off from the precursor web, the stretched leg elastic elements are simultaneously severed and will thereby snap back from the waist edges within the areas of the articles which are free from bonds between the leg elastic elements and the topsheet and backsheet webs. The snap-back portions of the leg elastic elements remain in the article as retracted, non-attached ends at the front and/or the rear portion of the absorbent article. However, it has been found that the application of leg elastic elements, in particular when the leg elastic involves snap-back portions, the provision of functional snap-back portions may negatively interfere with the attachment of other elements such as side flaps. In order to provide adequate snap-back for the elastic elements, it has been necessary to arrange the leg elastic elements and the bond-free snap-back areas of the article laterally inward of the side flap anchoring areas. In addition, in order to provide sufficient anchoring area for the side flaps, the portions of the topsheet and the backsheet which are arranged laterally outside of the absorbent core must be sufficiently wide to accommodate both the bond-free snap-back areas and the side flap anchoring areas side-by-side. This requirement has led to severe restrictions in the design of absorbent articles of this kind, in that either the topsheet and the backsheet have to be designed with a greater width than desired, or the absorbent core has to be designed with a smaller width than desired.

Accordingly, there exists a need for an elasticated absorbent article having an improved construction allowing a width of the absorbent core and the topsheet and backsheet to be freely selected and adapted to functional requirements of the absorbent article.

### SUMMARY

Disposable absorbent articles having good functionality, and which may be provided with functional leg elastic independently of other components such as side flaps, are achieved at least in part by the features of claim 1. Variations of the disclosure are set out in the dependent claims.

Disclosed herein is an absorbent article having first and second waist edges and first and second side edges, the front and rear waist edges of the absorbent article extending in a lateral direction of the absorbent article and the side edges extending in a longitudinal direction of the absorbent article. The absorbent article comprises
a topsheet,
a backsheet arrangement,
an absorbent member being disposed between the topsheet and the backsheet arrangement,
a first side flap extending from the first side edge in the lateral direction of the absorbent article and a second side flap extending from the second side edge in the lateral direction of the absorbent article, the first and second side flaps being attachment flaps, and
first and second leg elastic members extending in the longitudinal direction of the absorbent article along first and second side edges of the backsheet arrangement,
wherein the first and second side flaps each has an inner end portion which is secured between the topsheet and the backsheet arrangement within first and second flap anchoring areas being arranged at the first waist edge, each flap anchoring area having a lateral extension in the lateral direction of the absorbent article between an inner edge and an outer edge of the flap anchoring area and having a longitudinal extension in the longitudinal direction of the absorbent article between a waist edge of the side flap and a corresponding leg edge of the side flap.

The backsheet arrangement comprises first and second leg elastic anchoring portions extending along the side edges of the absorbent article, the first and second leg elastic anchoring portions comprising two layers of backsheet material, the first and second leg elastic members being arranged between the two layers of backsheet material, each leg elastic anchoring portion being fully or partly arranged laterally outward of the inner edge of the corresponding flap anchoring area, the first and second leg elastic members terminating at a distance from the first waist edge, whereby first leg elastic free portions are arranged at the first waist edge.

The first and second leg elastic members may be attached in the first and second leg elastic anchoring portions in a tensioned state. Alternatively, the first and second leg elastic members may be of a kind which can be activated e.g. by heat treatment or mechanical working. Such leg elastic members may be attached in a non-tensioned state and may be subsequently activated within a selected portion or portions of the leg elastic members.

The first leg elastic free portions may be formed by the first and second leg elastic members being retracted from the first waist edge. Retraction of the leg elastic members from the first leg elastic free portions may be accomplished by means of bond-free or substantially bond-free snap-back channels which are arranged in the longitudinal direction of the absorbent article, in the first leg elastic free portions of the backsheet arrangement.

After retraction, the first and second leg elastic members have a length in the longitudinal direction of the absorbent article which is less than a length of the absorbent article

As disclosed herein, the leg elastic members are shielded in the leg elastic anchoring portions of the backsheet arrangement and are not in direct contact with the topsheet. Hence, the leg elastic elements are not directly bonded to the topsheet. Attachment of the leg elastic members does not interfere with the attachment of the side flaps. This means that any snap-back portions of the leg elastic members may be placed wherever desired, such as in portions of the backsheet arrangement which overlap with the flap anchoring areas, without negatively affecting secure anchoring of the side flaps between the topsheet and the backsheet.

Side flaps constituting attachment flaps which are tensioned when putting on the absorbent article need to be firmly anchored between the topsheet and the backsheet to ascertain that they do not come off the article when exposed to laterally applied pulling forces. The side flaps may be elastic or non-elastic as known in the art.

The provision of a backsheet arrangement as disclosed herein, removes the necessity in previous absorbent article of leaving bond free snap-back channels between the topsheet and the backsheet for allowing retraction of severed leg elastic elements. Such bond-free snap-back channels constitute weakened areas in the absorbent article which may tear when subjected to tensile forces applied perpendicular to the extension of the bond-free channels. The side flaps are used when putting on the absorbent article and may be in the form of fastening flaps or fastening belt parts. Fastening flaps are usually fastened onto a body portion of the absorbent article. If the fastening flaps are placed at the rear waist edge of the article, the fastening flaps are stretched out and brought forward and fastened on the front portion of the absorbent article. If the fastening flaps are placed at the front waist edge of the absorbent article, the fastening flaps are brought rearward and fastened on the rear portion of the absorbent article. In order to obtain a good fit of the absorbent article around the waist of the wearer, the fastening flaps are generally applied under tension to activate any laterally extending elastic elements, such as waist elastic, flap elastic, etc. The side flaps are commonly provided with fasteners such as mechanical fasteners or adhesive fasteners with a first fastener element placed on the side flap and a mating fastener element or fastener reception area placed on the body portion to which the side flaps are to be fastened. Fastening belt parts are stretched out similar to the fastening flaps and are usually fastened together at their outer ends. To this end, they may be provided with fastener elements, in analogy with the fastening flaps.

In the previously known absorbent articles, the bond-free snap-back channels are arranged immediately inward of side flap anchoring areas in the lateral direction of the absorbent article. When the side flaps are pulled apart and tensioned during application of the absorbent article on a wearer, the weak non-bonded snap-back channels are exposed to tensile stress which may cause the material in the absorbent article to rupture along the snap-back channel.

By means of the backsheet arrangement as disclosed herein, the need for snap-back channels between the topsheet and the backsheet has been completely eliminated. In the absorbent articles as disclosed herein, all areas of the topsheet which are in direct contact with the backsheet arrangement may be bonded to each other such that no weakened areas are created in the laminate which is formed by the topsheet and the backsheet arrangement. It may be preferred that the backsheet is attached to the topsheet in all overlapping portions at least outside the periphery of the absorbent member. Preferably, there is no bond-free area between the end edges of the absorbent member and the waist edges of the absorbent article. Furthermore, there are no bond-free channels which may constitute weakened portions acting as tear indications when putting on the absorbent article.

The provision of a backsheet arrangement as disclosed herein provides freedom to place leg elastic elements in any selected part of the length of the absorbent article and at any suitable lateral position in the article, without having to take into consideration the placement and anchoring of side flaps such as fastening flaps or fastening belt parts. Furthermore, the width of the absorbent article between the side edges, as well as the width of the absorbent member may be freely selected to meet the functional requirements of the absorbent article with regard to fit and absorbency as the snap-back channels for the leg elastic elements may be arranged overlapping with the anchoring areas for the side flaps and not side-by-side, as in previous absorbent articles.

The absorbent articles as disclosed herein may have a rectangular shape, for ease of manufacturing. However, the side edges of the absorbent article may be cut to provide curved leg edges. The leg elastic elements may be arranged straight along the leg edges or may be arranged in a curve shape regardless of whether the absorbent article has a rectangular shape or is formed with curved leg edges.

The two layers of backsheet material forming the leg elastic anchoring portions in the backsheet arrangement may be attached to each other with a first bonding pattern within the leg elastic anchoring portions and with a second bonding pattern within the leg elastic free portions, the first bonding pattern being an elastic anchoring pattern and the second bonding pattern being an elastic release pattern. The elastic anchoring pattern serves to firmly attach the leg elastic in a functional state, such as a stretched state in the backsheet arrangement. The elastic release pattern joins the layers of backsheet material to each other within the leg elastic free portions, but does not prohibit retraction of stretched leg elastic between the layers of backsheet material. Accordingly, the elastic release pattern allows severed stretched elastic to snap-back within the leg elastic free portion.

The first and second leg elastic members may overlap with the first and second flap anchoring areas within 0% to 20% of the length of the first and second flap anchoring areas, preferably within 0% to 10% of the length of the first and second flap anchoring areas. The leg elastic members may thus terminate outside of the first and second flap anchoring areas, or may extend slightly in beneath the first and second flap anchoring areas.

The first and second leg elastic members may be any suitable leg elastic as known in the art, such as elastic bands, elastic strings, foam, etc. Each of the first and second leg elastic member may include multiple elastic strings or bands.

The topsheet may overlap with the leg elastic anchoring portions or may terminate inward of the leg elastic anchoring portions.

The two layers of backsheet material forming the leg elastic anchoring portions may be attached to each other by means of adhesive or by means of ultrasonic welding or by means of a combination of adhesive and ultrasonic welding.

The backsheet arrangement may comprise an inner layer and an outer layer. The inner and outer layers may be generally coextensive or the inner layer may have a greater width than the outer layer or vice versa.

The inner layer of the backsheet arrangement may be a barrier layer and the outer layer of the backsheet arrangement may be a nonwoven layer.

The outer layer of the backsheet arrangement may include a spunbond material and optionally a meltblown material.

The leg elastic anchoring portions may be formed by folded-in edge portions of the backsheet arrangement with the leg elastic members placed within the folds. A configuration of this type may be advantageous, as the folds may form softer and more cushioning leg edges than a corresponding leg edge formed by cut edges of laminated layers.

The leg elastic anchoring portions may be formed by laminating a first sheet of backsheet material with a second sheet of backsheet material or by laminating a first sheet of backsheet material with first and second strips of backsheet material. Such configuration may be advantageous as it allows different materials to be selected for the outer and the inner surfaces of the elastic anchoring portions.

The backsheet arrangement may comprise a laminate of a polymeric film and a fibrous nonwoven sheet. The fibrous nonwoven sheet may provide a textile outer surface or outer cover of the absorbent article and/or a soft, body contacting surface along the leg elastic members. The polymeric film may provide liquid barrier properties. The polymeric film may be any suitable liquid barrier film, as known in the art, such as a breathable liquid barrier film.

A polymeric film layer may be laminated to a nonwoven layer or to nonwoven strips having edge folds in which elastic elements have been applied. The polymeric film may be applied such that it does not extend all the way to side edges of a backsheet arrangement being defined by edge folds in a fibrous nonwoven layer.

Alternatively, a nonwoven layer may be folded in over a surface of a polymeric film layer to cover cut edges of the polymeric film layer.

The nonwoven sheet in the laminate may have a width in the lateral direction of the absorbent article which is greater than a width of the polymeric film in the lateral direction of the absorbent article. Hence, the nonwoven sheet may extend laterally beyond the polymeric film when in a planar state. The leg elastic anchoring portions may be formed completely by folds in the portions of the nonwoven sheet which extend outside the polymeric film. Such leg elastic anchoring portions are soft, pliable and highly breathable. Alternatively, the portions of the nonwoven sheet which extends laterally outside the polymeric film may be folded in over the polymeric film, so that the leg elastic anchoring portions comprise both the polymeric film and the nonwoven layer.

As disclosed herein, the leg elastic anchoring portions may be formed solely by fibrous nonwoven material, either as a folded portion in a layer of nonwoven material or as a laminate of two separate layers of nonwoven material. Fibrous nonwoven elastic anchoring portions provide the leg elastic with good breathability, softness and cushioning ability.

In an absorbent article as disclosed herein a third side flap may extend from the first side edge in the lateral direction of the article and a fourth side flap may extend from the second side edge in the lateral direction of the article, the third and fourth side flaps being arranged at the second waist edge. The third and fourth side flaps may be attachment flaps being configured to cooperate with the first and second attachment flaps which are arranged at the first waist edge.

The third and fourth side flaps may each have an inner end portion which is firmly attached between the topsheet and the backsheet arrangement within third and fourth flap anchoring areas, each flap anchoring area having a lateral extension in the lateral direction of the absorbent article between an inner edge and an outer edge of the flap anchoring area and having a longitudinal extension in the longitudinal direction of the absorbent article between a waist edge of the side flap and a corresponding leg edge of the side flap.

The leg elastic anchoring portions may be fully or partly arranged laterally outward of the corresponding inner edges of the third and fourth flap anchoring areas.

The first and second leg elastic members may terminate at a distance from the second waist edge, whereby second leg elastic free portions are arranged at the second waist edge.

The second leg elastic free portions may be formed by the first and second leg elastic members being retracted from the second waist edge.

The backsheet arrangement may be non-extensible in the lateral direction and/or in the longitudinal direction of the absorbent article. Alternatively, the backsheet arrangement may be extensible or elastically extensible in the lateral direction and/or in the longitudinal direction of the absorbent article. It may be desirable that the backsheet arrangement is non-extensible in the lateral direction of the absorbent article, as this may facilitate fitting of the absorbent article around the waist of a wearer by means of the first and second side flaps. Furthermore, it may be advantageous to be able to use a non-extensible backsheet for cost-saving reasons.

The first waist edge may be a rear waist edge and the second waist edge may be a front waist edge. Hence, the first and second side flaps are attachment flaps arranged at the rear waist edge of the absorbent article and being configured for fastening on the front portion of the absorbent article.

Alternatively, the first waist edge may be a front waist edge and the second waist edge may be a rear waist edge. With this configuration the first and second side flaps are attachment flaps arranged at the rear waist edge of the absorbent article and being configured for fastening on the rear portion of the absorbent article.

A fastener may be arranged on least one of the attachment flaps. It may be preferred that attachment flaps being provided with fasteners are arranged at the rear waist edge. When putting on the absorbent article on a wearer, the attachment flaps are stretched laterally and upwardly around the waist of the wearer. When both attachment flaps are provided with fasteners, the fasteners are generally fastened to a reception area on the outside of the article at the end of the article which is opposite to the attachment flaps. When only one attachment flap is provided with a fastener, the attachment flaps may be configured to be attached to each other in the manner of a belt.

The fasteners may be any type of fastener as known in the art, such as hook-type fasteners which are configured for being attached on a reception surface, such as on a surface having multiple loops. The reception surface may be an outer nonwoven layer of the backsheet arrangement or may be a particular loop material applied on an outer surface of the absorbent article. Alternatively the fasteners may be attached to attachment flaps arranged at the opposite end of the absorbent article.

### BRIEF DESCRIPTION OF THE DRAWINGS

The absorbent articles as disclosed herein will be further explained hereinafter with reference to the appended drawings wherein:
- Figure 1: shows a planar view of an absorbent article in the form of a diaper, from the side which is intended to be facing a wearer during use of the diaper;
- Figure 2: shows a cross-section through the diaper in Fig. 1, taken along the line II-II in Fig. 1;
- Figure 3: shows a corner portion of the absorbent article in Fig. 1 as indicated at III in Fig. 1;
- Figure 4: shows the absorbent article in Fig. 1 during application to a wearer;
- Figures 5a-5j: show cross-sections of different backsheet arrangements; and
- Figure 6: shows a detail of a backsheet arrangement comprising an elastic anchoring portion and an elastic free portion with different bonding patterns.

### DETAILED DESCRIPTION

Different aspects of the present disclosure will be described more fully hereinafter with reference to the enclosed drawings. The embodiments disclosed herein can, however, be realized in many different forms and should not be construed as being limited to the aspects set forth herein.

It is to be understood that the drawings are schematic and that individual components, such as layers of material are not necessarily drawn to scale.

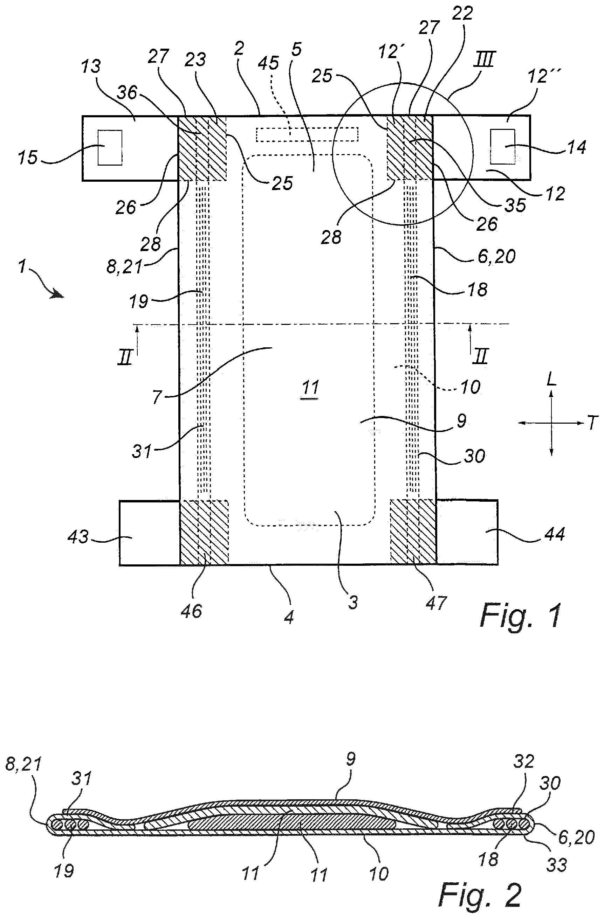

With initial reference to Figs. 1, 2, 3 and 4, there is shown an absorbent article 1 in the form of a diaper.

The absorbent article 1 has a first waist edge 2 and a second waist edge 4 and first and second side edges 6, 8. The first waist edge 2 of the absorbent article 1 which is shown in Fig. 1 is a rear waist edge and the second waist edge 4 of the absorbent article 1 is a front waist edge. The first and second waist edges 2, 4 extend in a lateral direction T of the absorbent article 1 and the side edges 6,8 extend in a longitudinal direction L of the absorbent article 1.

The absorbent article 1 which is shown in Figs. 1-4 comprises a topsheet 9 and a backsheet arrangement 10. An absorbent member 11 is disposed between the topsheet 9 and the backsheet arrangement 10. The absorbent article 1 may be divided in the longitudinal direction in a front portion 3, a rear portion 5 and a crotch portion 7 located between the front portion 3 and the rear portion 5.

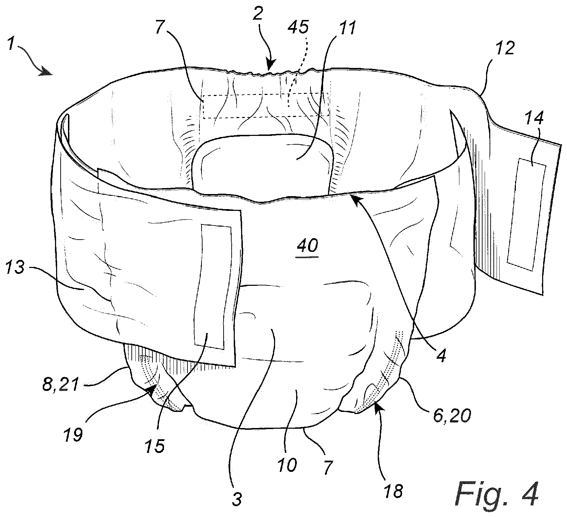

A first side flap 12 is arranged extending from the first side edge 6 in the lateral direction T of the absorbent article 1 and a second side flap 13 extends from the second side edge 8 in the lateral direction of the absorbent article. The first and second side flaps 12, 13 are attachment flaps and are provided with fasteners 14, 15 for securing the absorbent article 1 in a pant-like configuration as shown in Fig. 4.

First and second leg elastic members 18, 19 extend in the longitudinal direction L of the absorbent article along first and second side edges 20, 21 of the backsheet arrangement 10. In the absorbent article 1 which is shown in Figs. 1-4, the side edges of the backsheet arrangement 20, 21 coincide with the side edges 6, 8 of the absorbent article 1 and the end edges 2,4 of the backsheet arrangement 10 coincide with the end edges 2,4 of the absorbent article 1. It is not a prerequisite of the absorbent articles as disclosed herein that the end and side edges of the backsheet arrangement coincide with the end and side edges of the absorbent article.

The first and second side flaps 12, 13 each has an inner end portion 12', 13' which is secured between the topsheet 9 and the backsheet arrangement 10 within first and second flap anchoring areas 22, 23. The first and second flap anchoring areas 22, 23 are arranged at the first waist edge 2. Each flap anchoring area 22, 23 has a lateral extension in the lateral direction T of the absorbent article 1 between an inner edge 25 and an outer edge 26 of the flap anchoring area 22, 23 and has a longitudinal extension in the longitudinal direction L of the absorbent article 1 between a waist edge 27 of the side flap 12, 13 and a corresponding leg edge 28 of the side flap 12, 13. In the absorbent article 1 which is shown in Figs. 1-4, the waist edges 27 of the first and second side flaps 12, 13 coincide with the first waist edge 2, i.e. the rear waist edge of the absorbent article 1.

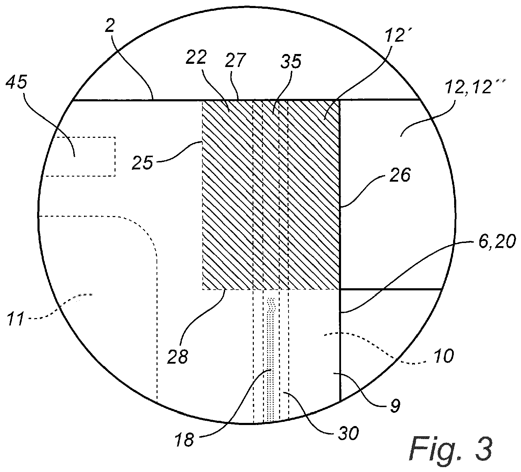

The backsheet arrangement 10 comprises first and second leg elastic anchoring portions 30, 31 extending along the side edges 6, 8 of the absorbent article. The first and second leg elastic anchoring portions 30, 31 each comprise two layers of backsheet material 32, 33, the first and second leg elastic members 18, 19 being arranged between the two layers of backsheet material 32, 33. In Figs. 1 and 3, the leg elastic anchoring portions 30, 31 and the leg elastic members 18, 19 are shown arranged laterally completely outward of the inner edges 25 of the flap anchoring areas 22, 23. The first and second leg elastic members 18, 19 terminate at a distance from the first waist edge 2, whereby first leg elastic free portions 35, 36 are arranged at the first waist edge 2. The first leg elastic free portions 35, 36 are arranged between the two layers of backsheet material 32, 33 and are placed at the ends of the leg elastic members 18, 19 within the areas of the first and second flap anchoring areas 22, 23.

The first and second leg elastic members 18, 19 are attached in the first and second leg elastic anchoring portions 30, 31 in a tensioned state. During production of the absorbent article 1, the leg elastic members 18, 19 have been bonded to the backsheet arrangement 10 such that they stay bonded within the leg elastic anchoring portions 30, 31 also after severing of the leg elastic members 18, 19. The portions of the leg elastic members 18, 19 which during production extend outside the leg elastic anchoring portions 30, 31 remain non-bonded or only weakly bonded during production and will snap-back from the first waist edge 2 when the absorbent article 1 is finally severed from a production web. Accordingly, the first leg elastic free portions 35, 36 are formed by means of snap-back channels which are formed at the waist edges 2, 4 of the absorbent article 1.

After retraction, the first and second leg elastic members 18, 19 have a length in the longitudinal direction L of the absorbent article 1 which is less than a total length of the absorbent article 1.

As disclosed herein, the leg elastic members 18, 19 are shielded in the leg elastic anchoring portions 30, 31 of the backsheet arrangement and are not in direct contact with the topsheet 9. Hence, the leg elastic members 18, 19 are not directly bonded to the topsheet 9. Attachment of the leg elastic members 18, 19 is made in a different plane from the attachment plane between the topsheet 9 and the backsheet arrangement 10 and does not interfere with the attachment of the side flaps 12, 13 as the bond-free or substantially bond-free snap-back channels provided in the elastic free areas are arranged in a different plane from the plane of the first and second flap anchoring areas 22, 23. In an absorbent article as disclosed herein, the need for snap-back channels between the topsheet and the backsheet has been completely eliminated. In the absorbent articles as disclosed herein, all areas of the topsheet which are in direct contact with the backsheet arrangement may be bonded to each other such that no weakened areas are created in the laminate which is formed by the topsheet and the backsheet arrangement. It may be preferred that the backsheet is attached to the topsheet in all overlapping portions at least outside the periphery of the absorbent member. Preferably, there is no bond-free area between the end edges of the absorbent member and the waist edges of the absorbent article. Furthermore, there are no bond-free channels which may constitute weakened portions acting as tear indications when putting on the absorbent article.

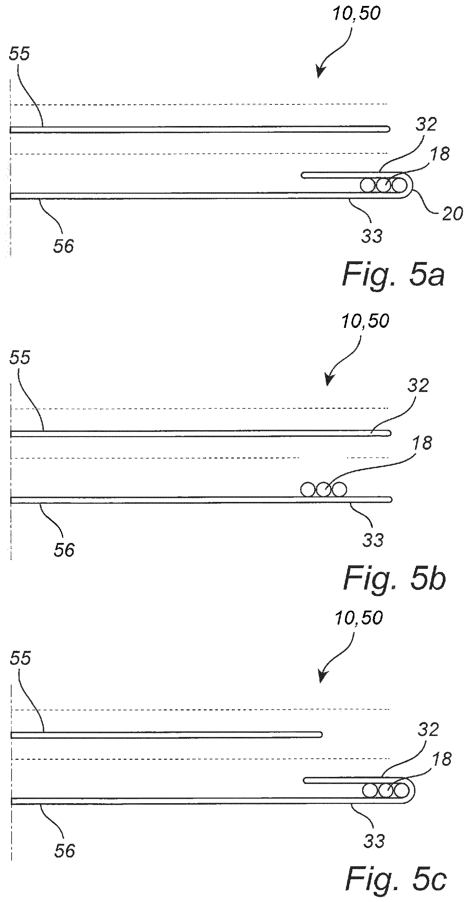

In the absorbent article 1 which is shown in Figs. 1-4, the leg elastic anchoring portions 30, 31 are formed by folded-in edge portions of the backsheet arrangement 10, as is seen in Fig. 2. The leg elastic anchoring portions may be formed in the backsheet arrangement 10 in any other way as disclosed herein as long as the leg elastic elements are attached between two layers of the backsheet arrangement. Some alternative configurations of the leg elastic anchoring portions are those shown in Figs. 5a - 5j.

The first and second side flaps 12, 13 are attachment flaps which are tensioned when putting on the absorbent article and need to be firmly anchored between the topsheet 9 and the backsheet 10 to ascertain that they do not come off the article when exposed to laterally applied pulling forces. The first and second side flaps 12, 13 may be made from any suitable material as known in the art, such as fibrous nonwoven material. The first and second side flaps 12, 13 may be elastic or may be elastically stretchable, at least in the lateral direction of the absorbent article 1. The first and second side flaps 12, 13 may be breathable and are preferably soft and pliable for providing good body conformance and comfort.

The first and second side flaps 12, 13 which are shown in Figs. 1-4 are provided with fastener elements 14, 15 which may be of any suitable kind such as adhesive or mechanical fasteners. A common type of mechanical fastener is hook-and-loop fasteners. In the absorbent article 1 in Figs. 1-4, the fastener elements 14, 15 may be seen as hook-type fasteners, i.e. fasteners having multiple protruding elements which may engage with a corresponding loop-type fastener such as a loop-type nonwoven material. The receiving area 40 is shown in fig. 4 to be located on the outside of the front portion 3 of the absorbent article 1, which in this case is an outer nonwoven layer of the backsheet arrangement 10. Alternatively, the fastener elements 14, 15 may be seen as adhesive fastener tapes which are arranged to be attached on a receiving area arranged on the front portion 3 of the absorbent article 1. As disclosed herein, the first and second side flaps 12, 13 may alternatively be arranged to be attached to third and fourth side flaps arranged at the opposite waist edge. Accordingly, the first and second side flaps 12, 13 which are arranged at the rear waist edge 2, may be configured to be attached to the third and fourth side flaps 43, 44 which are located at the front waist edge 4. Furthermore, the first and second side flaps 12, 13 may have a lateral extension which is large enough such that the first and second side flaps 12, 13 can be fastened to each other in the manner of a belt. It is to be understood that in a hook-and-loop type fastener arrangement, the hook-type fastener elements may be arranged on a receiving surface while the loop type fastener elements are arranged on a first and/or a second side flap.

The provision of a backsheet arrangement 10 as disclosed herein makes it possible to provide selected leg elastification while avoiding having snap-back channels formed by bond-free areas between the topsheet and the backsheet. Such bond-free snap-back channels constitute weakened areas in the absorbent article 1 which may tear when subjected to tensile forces applied perpendicular to the extension of the bond-free channels as the first and second side flaps 12, 13 are stretched out and brought forward and fastened on the front portion 3 of the absorbent article 1 under tension as illustrated in Fig. 4.

In previously known absorbent articles, bond-free snap-back channels are arranged immediately inward of side flap anchoring areas in the lateral direction of the absorbent article in order to avoid interference with the fastening of the side flaps. When the prior art side flaps are pulled apart and tensioned during application of the absorbent article on a wearer, the weak non-bonded snap-back channels are exposed to tensile stress which may cause the material in the absorbent article to rupture along the snap-back channel.

The third and fourth side flaps 43, 44 as shown in the figures are optional to the absorbent articles as disclosed herein. The third and fourth side flaps 43, 44, may be made from generally the same materials as the first and second side flaps 12, 13. The third and fourth side flaps 43, 44 may be provided with fastener elements, and may be arranged to contribute to fastening of the absorbent article on a wearer. In an absorbent article such as the absorbent article 1 in figs. 1-4 where the third and fourth side flaps 43, 44 are not fastening flaps, they will generally not be exposed to lateral tension and need not be as firmly anchored to the absorbent article as is the case for side flaps which are involved in tensioned fastening of the absorbent article around the waist of a wearer. Hence, in alternative to being attached between the topsheet 9 and the backsheet arrangement 10 in the same manner as the first and second side flaps 12,13, the third and fourth side flaps 43, 44 may be attached on the outer, wearer-facing surface of the topsheet 9 or on the outer, garment-facing surface of the backsheet arrangement 10.

When the third and fourth side flaps 43, 44 are attached between the topsheet 9 and the backsheet arrangement 10 in the same manner as the first and second side flaps 12,13, the leg elastic members 18, 19 are preferably applied in the same manner as in the rear portion 5 of the absorbent article 1, i.e. with elastic free portions 46, 47 created by arranging snap-back channels in the backsheet arrangement 10 at the front waist edge 4. The snap-back channels are arranged between the two layers of backsheet material 32, 33 between which the leg elastic members 18, 19 are applied.

The absorbent article 1 in Figs. 1-4 is further shown to include an optional waist elastic member 45. The waist elastic member 45 is located at the rear waist edge 2 and extends in the lateral direction along only a part of the rear waist edge 2. Any other type of waist elastic may be used, as known in the art.

The absorbent member 11 is disposed between the topsheet 9 and the backsheet arrangement 10 to absorb liquid, such as urine or other bodily fluids, which has passed through the topsheet 9. The absorbent member 11 may be a single-layer structure or may be a layered structure as shown in Fig. 2. The absorbent member 11 may comprise suitable amounts of superabsorbent material. Such superabsorbent material is well known in the field of absorbent articles, and is constituted by water-swellable and water-insoluble material which is capable of absorbing large quantities of fluid upon formation of a hydrogel. The absorbent component may contain superabsorbent material in the form of fibers or particles of absorbent polymer material. For example, the superabsorbent material may be surface cross-linked, partially neutralized polyacrylates. The superabsorbent material, e.g., the superabsorbent fibers or particles, may be mixed with other absorbent or liquid uptake material or materials, such as cellulose fluff pulp, and/or be arranged in pockets or layers in the absorbent member 11.

The absorbent member 11 may further comprise components for improving properties of the absorbent member 11, such as integrity and strength. For example, the absorbent member 11 may comprise a binder or binders, such as binder fibers. Resilient fibers, chemically stiffened fibers, etc. may be present in the absorbent member 11 to counteract wet-collapse of cellulosic fibers. Such fibers may also be useful in retaining a fluid transporting capillary network in the absorbent component so that absorbent fluid may be distributed in the absorbent component and be absorbed by superabsorbent material also in parts of the absorbent component outside the initial wetting area of the absorbent article.

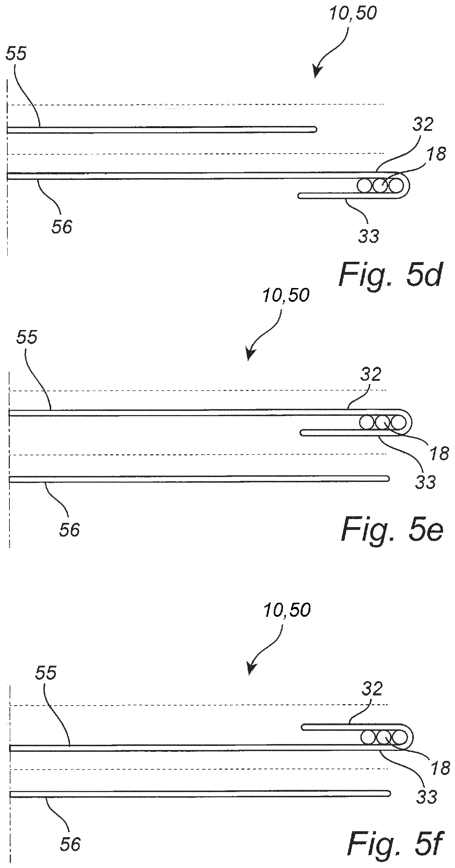

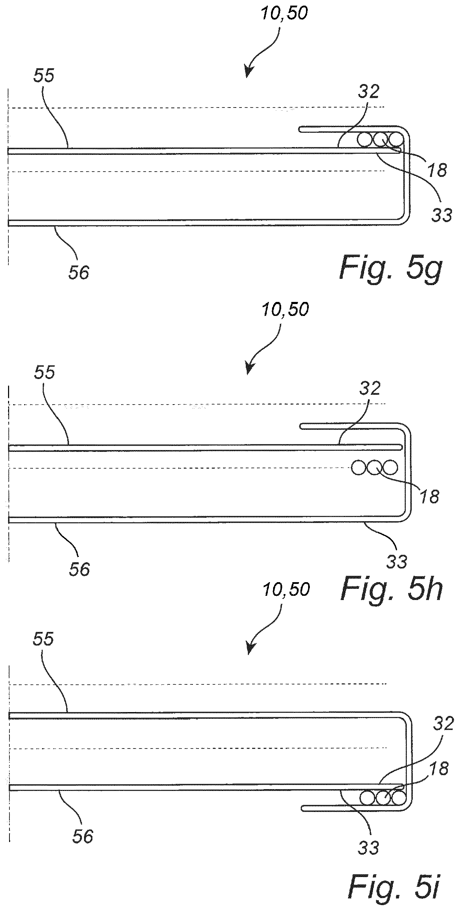

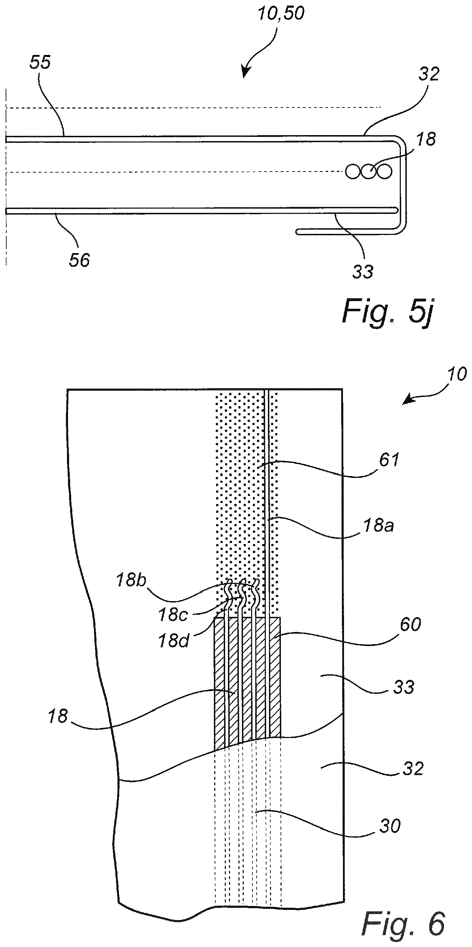

With reference to Fig. 5, there is shown a number of cross-sections through a leg elastic snap-back portion 50 of different backsheet arrangements 10 which may be used in any absorbent article as disclosed herein. The snap-back portions are shown as they appear during production of an absorbent article as disclosed herein, before severing of individual articles from the production web of interconnected articles, i.e. before the tensioned elastic members 18 have been cut off and retracted from the snap-back portion 50 to create a leg elastic free portion. The broken lines in all of Figs. 5a-5j indicate the presence of construction bonding such as construction adhesive, thermo-bonding or ultrasonic bonding. Construction bonding is used to bond the layers and components of the backsheet arrangements 10 together and to bond the backsheet arrangement to other components of the absorbent article such as a topsheet, side flaps, etc. The leg elastic anchoring portions of the backsheet arrangements in Figs. 5a-5j have the same appearance as the snap-back portions 50 shown in Figs. 5a-5j, except that construction bonding is present to anchor the leg elastic members 18 in the leg elastic anchoring portions.

All the backsheet arrangements 10 which are shown in Figs. 5a-5j comprise a first sheet material 55 and a second sheet material 56. The backsheet arrangements are intended to be incorporated into an absorbent article as disclosed herein with the first sheet material 55 being an inner layer facing toward the topsheet of the absorbent article and with the second sheet material 56 being an outer layer facing away from the topsheet of the absorbent article. The first sheet material 55 may be a liquid barrier material such as a liquid-impermeable plastic film, a nonwoven sheet which has been coated with a liquid barrier material, or some other flexible material sheet which has the ability to withstand liquid penetration. It may be advantageous if the liquid-impervious barrier is breathable, i.e. permits the passage of water vapour through the barrier sheet. The first sheet material 55 may be stretchable, elastically stretchable or non-elastic. The second sheet material 56, may be an outer covering sheet which may have a textile or textile-like appearance and/or at textile or textile-like feel. The second sheet material 56 may be a plastic film material, e.g. an embossed, reduced gloss film or a fibrous nonwoven material. Fibrous nonwoven materials have an excellent textile appearance and feel and low gloss and may be preferred as outer cover layers. However, it is to be understood that a backsheet arrangement as disclosed herein may consist of a single sheet material having the elastic elements arranged in side folds in the sheet material. Such backsheet arrangements would be accomplished e.g. by omitting the first sheet material 55 in Figs. 5a, 5c and 5d, and by omitting the second sheet material 56 in Figs. 5e and 5f.

Furthermore, although the first and second sheets of material are shown as continuous sheets, one of the first and second sheet materials may be a strips of backsheet material with a corresponding strip of backsheet material used in the leg elastic anchoring portion which is arranged along the opposite side edge of the backsheet arrangement.

In the backsheet arrangement which is shown in Fig. 5a, the elastic members 18 are arranged in an edge fold in the second sheet material 56, the second sheet material 56 forming both a first layer of backsheet material 32 and a second layer of backsheet material 33 between which two layers 32, 33 of backsheet material the leg elastic members 18 are applied. The edge-fold in the second sheet material 56 is made such that the folded-in edge portion 32 of the second sheet material 56 is located between the first sheet material 55 and the second sheet material 56. The first sheet material 55 is attached to the second sheet material 56 and is almost coextensive with the second sheet material in that the first sheet material 55 terminates in the lateral direction only a small distance from side edge 20 of the backsheet arrangement, the side edge 20 being defined by the edge fold in the second sheet material 56. In this manner, the first sheet material 55 extends in over the leg elastic members 18.

In the backsheet arrangement which is shown in Fig. 5b, the first sheet material 55 and the second sheet material 56 are non-folded and generally coextensive and form the first and second layers of backsheet material 32, 33 between which the leg elastic members 18 are arranged.

The backsheet arrangement which is shown in Fig. 5c differs from the backsheet arrangement 10 which is shown in Fig. 5a in that the first sheet material 55 extends laterally only slightly in over the folded-in edge portion of the second sheet material 56. Hence, the first sheet material 55 does not extend in over the elastic elements.

The backsheet arrangement which is shown in Fig. 5d differs from the backsheet arrangement 10 which is shown in Fig. 5c in that the edge-fold in the second sheet material 56 is made such that the folded-in edge portion of the second sheet material 56 is located on the side of the second sheet material 56 which faces away from the first sheet material 55.

In the backsheet arrangement which is shown in Fig. 5e, the leg elastic members 18 are arranged in an edge fold in the first sheet material 55, the first sheet material 55 forming both a first layer of backsheet material 32 and a second layer of backsheet material 33 between which two layers of backsheet material 32, 33 the leg elastic members 18 are applied. The edge-fold in the first sheet material 55 is made such that the folded-in edge portion 33 of the first sheet material 55 is located between the first sheet material 55 and the second sheet material 56. The second sheet material 56 is attached to the first sheet material 55 and is almost coextensive with the first sheet material in that the second sheet material 56 terminates in the lateral direction only a small distance from the side edge 20 of the backsheet arrangement 10, the side edge 20 being defined by the edge fold in the first sheet material 55. In this manner, the second sheet material 56 extends in over the leg elastic members 18.

The backsheet arrangement which is shown in Fig. 5f differs from the backsheet arrangement 10 which is shown in Fig. 5e in that the edge-fold in the first sheet material 55 is made such that the folded-in edge portion 32 of the first sheet material 55 is located on the side of the first sheet material 55 which faces away from the second sheet material 56.

In the backsheet arrangement which is shown in Fig. 5g, the leg elastic members 18 are arranged along a non-folded edge portion 33 of the first sheet material 55 on the surface of the first sheet material 55 which faces away from the second sheet material 56 and are covered by a folded-in edge portion 32 of the second sheet material 56, the folded-in edge portion 32 being folded onto the surface of the first sheet material 55 which faces away from the second sheet material 56.

The backsheet arrangement which is shown in Fig. 5h, differs from the backsheet arrangement which is shown in Fig. 5g in that the leg elastic members 18 are arranged on the surface of the first sheet material 55 which faces towards the second sheet material 56.

The backsheet arrangements which are shown in Figs. 5i and 5j, are upside-down variants of the backsheet arrangements which are shown in Figs. 5g and 5h. Accordingly, in Fig. 5i the leg elastic members 18 are arranged on the surface of the second sheet material 56 which faces away from the first sheet material 55 and are covered by a folded-over portion 33 of the first sheet material 55. In Fig. 5j, the leg elastic members 18 are arranged between the first and second sheet materials 55, 56.

With reference to Figs. 6, the two layers of backsheet material 32, 33 forming a leg elastic anchoring portion 30 may be attached to each other with a first bonding pattern 60 within the leg elastic anchoring portion 30 and with a second bonding pattern 61 within the leg elastic free portion 35. The first bonding pattern 60 is an elastic anchoring pattern and the second bonding pattern 61 is an elastic release pattern.

The elastic anchoring pattern 60 serves to firmly attach the elastic elements18a-d of the leg elastic member 18 in a stretched functional state in the backsheet arrangement 10. The elastic release pattern 61 joins the layers of backsheet material 32, 33 to each other within the leg elastic free portions 35, but does not prohibit retraction of tensioned leg elastic between the layers of backsheet material. Accordingly, the elastic release pattern allows severed stretched elastic to snap-back within the leg elastic free portion.

The two layers of backsheet material 32, 33 forming the leg elastic anchoring portion 30 and the elastic release portion 35 may be attached to each other by means of adhesive. The adhesive is then applied with a greater surface coverage and/or which a greater basis weight within the area of the elastic anchoring pattern 60 than within the area of the elastic release pattern 61. Bonding may alternatively be effected by means of heat welding or ultrasonic welding as set out in EP 3 056 176 A1 and US 2016/0058624 A1. A denser boding pattern is then created in the leg elastic anchoring portion 30 than in the elastic release portion 35. The sparsely dispersed bond points or the adhesive in the elastic release pattern 61 are insufficient to counteract the retractive forces acting on the tensioned elastic elements 18a-d when the elastic elements 18a-d are severed. Consequently, the severed elastic elements 18b-d are released from any bonds in the elastic release pattern 61 and snap back from the position illustrated by the elastic element 18a, towards the leg elastic anchoring portion 30, as illustrated by the elastic elements 18b-18d.

Bonding between the first sheet material 55 and the second sheet material 56 outside the elastic anchoring portion 30 and the elastic release portion 35 may be made with any suitable bond pattern and bonding method.

The elastic member 18 in Fig. 6 is shown as a multistring elastic member with four elastic elements 18a-18e. it is to be understood that any suitable number of elastic elements may be used, such as 1-30 elastic elements, 2-20 elastic elements or 5 to 15 elastic elements.

## Claims

1. An absorbent article (1) having first and second waist edges (2, 4) and first and second side edges (6, 8), the front and rear waist edges (2, 4) of the absorbent article (1) extending in a lateral direction (T) of the absorbent article (1) and the side edges (6, 8) extending in a longitudinal direction (L) of the absorbent article (1), the absorbent article (1) comprising
a topsheet (9),
a backsheet arrangement (10),
an absorbent member (11) being disposed between the topsheet (9) and the backsheet arrangement (10),
a first side flap (12) extending from the first side edge (6) in the lateral direction (T) of the absorbent article (1) and a second side flap (13) extending from the second side edge (8) in the lateral direction (T) of the absorbent article (1) the first and second side flaps (12, 13) being attachment flaps, and
first and second leg elastic members (18, 19) extending in the longitudinal direction (L) of the absorbent article (1) along first and second side edges (20, 21) of the backsheet arrangement (10),
wherein the first and second side flaps (13) each has an inner end portion (12', 13') which is secured between the topsheet (9) and the backsheet arrangement (10) within first and second flap anchoring areas (22, 23) being arranged at the first waist edge (2), each flap anchoring area (22, 23) having a lateral extension in the lateral direction (T) of the absorbent article (1) between an inner edge (25) and an outer edge (26) of the flap anchoring area (22, 23) and having a longitudinal extension in the longitudinal direction (L) of the absorbent article (1) between a waist edge (27) of the side flap (12, 13) and a corresponding leg edge (28) of the side flap (12, 13),
wherein the backsheet arrangement (10) comprises first and second leg elastic anchoring portions (30, 31) extending along the side edges (6, 8) of the absorbent article (1), the first and second leg elastic anchoring portions (30, 31) comprising two layers of backsheet material (32, 33), the first and second leg elastic members (18, 19) being arranged between the two layers of backsheet material (32, 33), each leg elastic anchoring portion (30, 31) being fully or partly arranged laterally outward of the inner edge (25) of the corresponding flap anchoring area (22, 23), the first and second leg elastic members (18, 19) terminating at a distance from the first waist edge (2), whereby first leg elastic free portions (35, 36) are arranged at the first waist edge (2).

2. An absorbent article (1) according to claim 1, wherein the first and second leg elastic members (18, 19) are attached in the first and second leg elastic anchoring portions (30, 31) in a tensioned state, and preferably, wherein the first leg elastic free portions (35, 36) are formed by the first and second leg elastic members (18, 19) being retracted from the first waist edge (2).

3. An absorbent article (1) according to any one of the preceding claims, wherein the two layers of backsheet material (32, 33) forming the leg elastic anchoring portions (30, 31) are attached to each other with a first bonding pattern (60) within the leg elastic anchoring portions (30, 31) and with a second bonding pattern (61) within the leg elastic free portions (35,36), the first bonding pattern (60) being an elastic anchoring pattern (60) and the second bonding pattern (61) being an elastic release pattern (61).

4. An absorbent article (1) according to any one of the preceding claims, wherein the first and second leg elastic members (18, 19) overlap with the first and second flap anchoring areas (22, 23) within 0% to 20% of the length of the first and second flap anchoring areas (22, 23), preferably within 0% to 10% of the length of the first and second flap anchoring areas (22, 23).

5. An absorbent article (1) according to any one of the preceding claims, wherein the first and second leg elastic members (18, 19) include multiple elastic strings.

6. An absorbent article (1) according to any one of the preceding claims, wherein the topsheet (9) overlaps with the leg elastic anchoring portions (30, 31).

7. An absorbent article (1) according to any one of the preceding claims, wherein the two layers of backsheet material (32, 33) forming the leg elastic anchoring portions (30, 31) are attached to each other by means of adhesive, or by means of ultrasonic welding.

8. An absorbent article (1) according to any one of the preceding claims, wherein the backsheet arrangement (10) comprises an inner layer and an outer layer.

9. An absorbent article (1) according to any one of the preceding claims, wherein the leg elastic anchoring portions (30, 31) are formed by folded-in edge portions of the backsheet arrangement (10), or wherein the leg elastic anchoring portions (30,31) are formed by laminating a first sheet of backsheet material with a second sheet of backsheet material or by laminating a first sheet of backsheet material with first and second strips of backsheet material.

10. An absorbent article (1) according any one of the preceding claims, wherein the backsheet arrangement (10) comprises a laminate of a polymeric film and a fibrous nonwoven sheet, and preferably wherein the nonwoven sheet has a width in the lateral direction (T) of the absorbent article (1) which is greater than a width of the polymeric film in the lateral direction (T) of the absorbent article (1).

11. An absorbent article (1) according to any one of the preceding claims, wherein the leg elastic anchoring portions (30, 31) are formed by fibrous nonwoven material.

12. An absorbent article (1) according to any one of the preceding claims, wherein a third side flap (43) extends from the first side edge (6) of the absorbent article (1) in the lateral direction (T) of the absorbent article (1) and a fourth side flap (44) extends from the second side edge (8) of the absorbent article (1) in the lateral direction of the article (1), the third and fourth side flaps (43, 44) being arranged at the second waist edge (4).

13. An absorbent article (1) according to claim 12, wherein the third and fourth side flaps (43, 44) each has an inner end portion which is firmly attached between the topsheet (9) and the backsheet arrangement (10) within third and fourth flap anchoring areas, each flap anchoring area having a lateral extension in the lateral direction of the absorbent article (1) between an inner edge and an outer edge of the flap anchoring area and having a longitudinal extension in the longitudinal direction (L) of the absorbent article (1) between the a waist edge of the side flap (43, 44) and a corresponding leg edge of the side flap (43, 44), and preferably wherein the leg elastic anchoring portions (30,31) are fully or partly arranged laterally outward of the corresponding inner edges of the third and fourth flap anchoring areas (43, 44).

14. An absorbent article (1) according to any one of claims 12 and 13, wherein the first and second leg elastic members (18, 19) terminate at a distance from the second waist edge (4), whereby second leg elastic free portions (46, 47) are arranged at the second waist edge (4), and optionally, wherein the second leg elastic free portions (46, 47) are formed by the first and second leg elastic members (18, 19) being retracted from the second waist edge (4).

15. An absorbent article (1) according to any one of the preceding claims, wherein the backsheet arrangement (10) is non-extensible in the lateral direction (T) of the absorbent article (1).

## Patentansprüche

1. Absorbierender Artikel (1) mit einem ersten und zweiten Taillenrand (2, 4) und einem ersten und zweiten Seitenrand (6, 8), wobei sich der vordere und hintere Taillenrand (2, 4) des absorbierenden Artikels (1) in eine seitliche Richtung (T) des absorbierenden Artikels (1) erstrecken und sich die Seitenränder (6, 8) in eine Längsrichtung (L) des absorbierenden Artikels (1) erstrecken, wobei der absorbierende Artikel (1) Folgendes umfasst
eine Oberschicht (9),
eine Unterschichtanordnung (10),
ein absorbierendes Element (11), das zwischen der Oberschicht (9) und der Unterschichtanordnung (10) angeordnet ist,
eine erste Seitenklappe (12), die sich von dem ersten Seitenrand (6) in die seitliche Richtung (T) des absorbierenden Artikels (1) erstreckt, und eine zweite Seitenklappe (13), die sich von dem zweiten Seitenrand (8) in die seitliche Richtung (T) des absorbierenden Artikels (1) erstreckt, wobei die erste und zweite Seitenklappe (12, 13) Befestigungsklappen sind, und
ein erstes und zweites elastisches Beinelement (18, 19), die sich in die Längsrichtung (L) des absorbierenden Artikels (1) entlang dem ersten und zweiten Seitenrand (20, 21) der Unterschichtanordnung (10) erstrecken,
wobei die erste und zweite Seitenklappe (13) jeweils ein inneres Endstück (12', 13') aufweisen, das zwischen der Oberschicht (9) und der Unterschichtanordnung (10) innerhalb des ersten und zweiten Klappenverankerungsbereichs (22, 23) befestigt ist, die an dem ersten Taillenrand (2) angeordnet sind, wobei jeder Klappenverankerungsbereich (22, 23) eine seitliche Ausdehnung in die seitliche Richtung (T) des absorbierenden Artikels (1) zwischen einem Innenrand (25) und einem Außenrand (26) des Klappenverankerungsbereichs (22, 23) aufweist und eine Längsausdehnung in die Längsrichtung (L) des absorbierenden Artikels (1) zwischen einem Taillenrand (27) der Seitenklappe (12, 13) und einem entsprechenden Beinrand (28) der Seitenklappe (12, 13) aufweist,
wobei die Unterschichtanordnung (10) einen ersten und zweiten elastischen Beinverankerungsteil (30, 31) umfasst, die sich entlang der Seitenränder (6, 8) des absorbierenden Artikels (1) erstrecken, das erste und zweite elastische Beinverankerungsteil (30, 31) zwei Schichten des Unterschichtmaterials (32, 33) umfassen, das erste und zweite elastische Beinelement (18, 19) zwischen den zwei Schichten des Unterschichtmaterials (32, 33) angeordnet sind, jeder elastische Beinverankerungsteil (30, 31) vollständig oder teilweise seitlich außerhalb des Innenrandes (25) des entsprechenden Klappenverankerungsbereichs (22, 23) angeordnet ist, das erste und zweite elastische Beinelement (18, 19) in einem Abstand von dem ersten Taillenrand (2) enden, wobei erste elastikfreie Beinteile (35, 36) an dem ersten Taillenrand (2) angeordnet sind.

2. Absorbierender Artikel (1) nach Anspruch 1, wobei das erste und zweite elastische Beinelement (18, 19) in dem ersten und zweiten elastischen Beinverankerungsteil (30, 31) in einem gespannten Zustand angebracht sind, und wobei vorzugsweise die ersten elastikfreien Beinteile (35, 36) dadurch gebildet sind, dass das erste und zweite elastische Beinelement (18, 19) von dem ersten Taillenrand (2) zurückgezogen sind.

3. Absorbierender Artikel (1) nach einem der vorhergehenden Ansprüche, wobei die zwei Schichten des Unterschichtmaterials (32, 33), die die elastischen Beinverankerungsteile (30, 31) bilden, mit einem ersten Verbindungsmuster (60) innerhalb der elastischen Beinverankerungsteile (30, 31) und mit einem zweiten Verbindungsmuster (61) innerhalb der elastikfreien Beinteile (35, 36) aneinander befestigt sind, wobei das erste Verbindungsmuster (60) ein elastisches Verankerungsmuster (60) ist und das zweite Verbindungsmuster (61) ein elastisches Freigabemuster (61) ist.

4. Absorbierender Artikel (1) nach einem der vorhergehenden Ansprüche, wobei das erste und zweite elastische Beinelement (18, 19) den ersten und zweiten Klappenverankerungsbereich (22, 23) innerhalb von 0 % bis 20 % der Länge des ersten und zweiten Klappenverankerungsbereichs (22, 23) überlappen, vorzugsweise innerhalb von 0 % bis 10 % der Länge des ersten und zweiten Klappenverankerungsbereichs (22, 23).

5. Absorbierender Artikel (1) nach einem der vorhergehenden Ansprüche, wobei das erste und zweite elastische Beinelement (18, 19) mehrere elastische Schnüre enthalten.

6. Absorbierender Artikel (1) nach einem der vorhergehenden Ansprüche, wobei die Oberschicht (9) die elastischen Beinverankerungsteile (30, 31) überlappt.

7. Absorbierender Artikel (1) nach einem der vorhergehenden Ansprüche, wobei die zwei Schichten des Oberschichtmaterials (32, 33), die die elastischen Beinverankerungsteile (30, 31) bilden, durch Klebstoff oder durch Ultraschallschweißen aneinander befestigt sind.

8. Absorbierender Artikel (1) nach einem der vorhergehenden Ansprüche, wobei die Unterschichtanordnung (10) eine Innenschicht und eine Außenschicht umfasst.

9. Absorbierender Artikel (1) nach einem der vorhergehenden Ansprüche, wobei die elastischen Beinverankerungsteile (30, 31) durch eingefaltete Randteile der Unterschichtanordnung (10) gebildet sind oder wobei die elastischen Beinverankerungsteile (30,31) durch Laminieren einer ersten Schicht des Unterschichtmaterials mit einer zweiten Schicht des Unterschichtmaterials oder durch Laminieren einer ersten Schicht des Unterschichtmaterials mit ersten und zweiten Streifen des Unterschichtmaterials gebildet sind.

10. Absorbierender Artikel (1) nach einem der vorhergehenden Ansprüche, wobei die Unterschichtanordnung (10) ein Laminat aus einer Polymerfolie und einer faserigen Vliesschicht umfasst, und wobei vorzugsweise die Vliesschicht eine Breite in die seitliche Richtung (T) des absorbierenden Artikels (1) aufweist, die größer als eine Breite der Polymerfolie in die seitliche Richtung (T) des absorbierenden Artikels (1) ist.

11. Absorbierender Artikel (1) nach einem der vorhergehenden Ansprüche, wobei die elastischen Beinverankerungsteile (30, 31) aus faserigem Vliesmaterial gebildet sind.

12. Absorbierender Artikel (1) nach einem der vorhergehenden Ansprüche, wobei sich eine dritte Seitenklappe (43) von dem ersten Seitenrand (6) des absorbierenden Artikels (1) in die seitliche Richtung (T) des absorbierenden Artikels (1) erstreckt und sich eine vierte Seitenklappe (44) von dem zweiten Seitenrand (8) des absorbierenden Artikels (1) in die seitliche Richtung des Artikels (1) erstreckt, wobei die dritte und vierte Seitenklappe (43, 44) an dem zweiten Taillenrand (4) angeordnet sind.

13. Absorbierender Artikel (1) nach Anspruch 12, wobei die dritte und vierte Seitenklappe (43, 44) jeweils ein inneres Endstück aufweisen, das zwischen der Oberschicht (9) und der Unterschichtanordnung (10) innerhalb des dritten und vierten Klappenverankerungsbereichs fest angebracht ist, wobei jeder Klappenverankerungsbereich eine seitliche Ausdehnung in die seitliche Richtung des absorbierenden Artikels (1) zwischen einem Innenrand und einem Außenrand des Klappenverankerungsbereichs aufweist und eine Längsausdehnung in die Längsrichtung (L) des absorbierenden Artikels (1) zwischen dem Taillenrand der Seitenklappe (43, 44) und einem entsprechenden Beinrand der Seitenklappe (43, 44) aufweist, und wobei vorzugsweise die elastischen Beinverankerungsteile (30,31) vollständig oder teilweise seitlich außerhalb der entsprechenden Innenränder des dritten und vierten Klappenverankerungsbereichs (43, 44) angeordnet sind.

14. Absorbierender Artikel (1) nach einem der Ansprüche 12 und 13, wobei das erste und zweite elastische Beinelement (18, 19) in einem Abstand von dem zweiten Taillenrand (4) enden, wobei zweite elastikfreie Beinteile (46, 47) an dem zweiten Taillenrand (4) angeordnet sind und wobei möglicherweise die zweiten elastikfreien Beinteile (46, 47) dadurch gebildet sind, dass das erste und zweite elastische Beinelement (18, 19) von dem zweiten Taillenrand (4) zurückgezogen sind.

15. Absorbierender Artikel (1) nach einem der vorhergehenden Ansprüche, wobei die Unterschichtanordnung (10) in die seitliche Richtung (T) des absorbierenden Artikels (1) nicht dehnbar ist.

## Revendications

1. Article absorbant (1) ayant un premier et deuxième bords de taille (2, 4) et un premier et deuxième bords latéraux (6, 8), les bords de taille avant et arrière (2, 4) de l'article absorbant (1) s'étendant dans une direction latérale (T) de l'article absorbant (1) et les bords latéraux (6, 8) s'étendant dans une direction longitudinale (L) de l'article absorbant (1), l'article absorbant (1) comprenant
une feuille supérieure (9),
un agencement de feuille arrière (10),
un élément absorbant (11) étant disposé entre la feuille supérieure (9) et l'agencement de feuille arrière (10),
un premier rabat latéral (12) s'étendant du premier bord latéral (6) dans la direction latérale (1) de l'article absorbant (1) et un deuxième rabat latéral (13) s'étendant du deuxième bord latéral (8) dans la direction latérale (T) de l'article absorbant (1) le premier et deuxième rabats latéraux (12, 13) étant des rabats de fixation, et
le premier et deuxième éléments élastiques de jambe (18, 19) s'étendant dans la direction longitudinale (L) de l'article absorbant (1) le long d'un premier et deuxième bords latéraux (20, 21) de l'agencement de feuille arrière (10),
où le première et deuxième rabats latéraux (13) ont chacun une portion d'extrémité intérieure (12', 13') qui est sécurisée entre la feuille supérieure (9) et l'agencement de feuille arrière (10) dans une première et deuxième zones d'ancrage de rabat (22, 23) étant disposées au premier bord de taille (2), chaque zone d'ancrage de rabat (22, 23) ayant une extension latérale dans la direction latérale (T) de l'article absorbant (1) entre un bord intérieur (25) et un bord extérieur (26) de la zone d'ancrage de rabat (22, 23) et ayant une extension longitudinale dans la direction longitudinale (L) de l'article absorbant (1) entre un bord de taille (27) du rabat latéral (12, 13) et un bord de jambe correspondant (28) du rabat latéral (12, 13),
où l'agencement de feuille arrière (10) comprend une première et deuxième portions d'ancrage élastique de jambe (30, 31) s'étendant le long des bords latéraux (6, 8) de l'article absorbant (1), la première et deuxième portions d'ancrage élastique de jambe (30, 31) comprenant deux couches de matériau de feuille arrière (32, 33), le premier et deuxième éléments élastiques de jambe (18, 19) étant arrangés entre les deux couches de matériau de feuille arrière (32, 33), chaque portion d'ancrage élastique de jambe (30, 31) étant complètement ou partiellement disposée latéralement à l'extérieur du bord intérieur (25) de la zone d'ancrage de rabat correspondante (22, 23), le premier et deuxième éléments élastiques de jambe (18, 19) terminant à une distance du premier bord de taille (2), de sorte que des premières portions de jambe libres d'élastique (35, 36) sont disposées au premier bord de taille (2).

2. Article absorbant (1) selon la revendication 1, où le premier et deuxième éléments élastiques de jambe (18, 19) sont attachés à la première et deuxième portions d'ancrage élastique de jambe (30, 31) dans un état tendu, et de préférence, où les premières portions de jambe libres d'élastique (35, 36) sont formées par le premier et deuxième éléments élastique de jambe (18, 19) étant rétractés à partir du premier bord de taille (2).

3. Article absorbant (1) selon l'une quelconque des revendications précédentes, où les deux couches de matériau de feuille arrière (32, 33) formant des portions d'ancrage élastique de jambe (30, 31) sont attachées l'une à l'autre avec un premier modèle de liaison (60) dans les portions d'ancrage élastique de jambe (30, 31) et avec un deuxième modèle de liaison (61) dans les portions de jambe libre d'élastique (35,36), le premier modèle de liaison (60) étant un modèle d'ancrage élastique (60) et le deuxième modèle de liaison (61) étant un modèle de relâchement élastique (61).

4. Article absorbant (1) selon l'une quelconque des revendications précédentes, où le premier et deuxième éléments élastiques de jambe (18, 19) se chevauchent avec la première et deuxième zones d'ancrage de rabat (22, 23) de 0% à 20% de la longueur de la première et deuxième zones d'ancrage de rabat (22, 23), de préférence de 0% à 10% de la longueur de la première et deuxième zones d'ancrage de rabat (22, 23).

5. Article absorbant (1) selon l'une quelconque des revendications précédentes, où le premier et deuxième éléments élastiques de jambe (18, 19) comprennent de multiples cordes élastiques.

6. Article absorbant (1) selon l'une quelconque des revendications précédentes, où la feuille supérieure (9) chevauche les portions d'ancrage élastique de jambe (30, 31).

7. Article absorbant (1) selon l'une quelconque des revendications précédentes, où les deux couches de matériau de feuille arrière (32, 33) formant les portions d'ancrage élastique de jambe (30, 31) sont attachées l'une à l'autre au moyen d'adhésif, ou au moyen de soudage par ultrasons.

8. Article absorbant (1) selon l'une quelconque des revendications précédentes, où l'agencement de feuille arrière (10) comprend une couche intérieure et une couche extérieure.

9. Article absorbant (1) selon l'une quelconque des revendications précédentes, où les portions d'ancrage élastique de jambe (30, 31) sont formées par des portions de bord repliées de l'agencement de feuille arrière (10), ou où les portions d'ancrage élastique de jambe (30,31) sont formées en laminant une première feuille de matériau de feuille arrière avec une deuxième feuille de matériau de feuille arrière ou en laminant une première feuille de matériau de feuille arrière avec une première et deuxième bandes de matériau de feuille arrière.

10. Article absorbant (1) selon l'une quelconque des revendications précédentes, où l'agencement de feuille arrière (10) comprend un stratifié d'un film polymérique et une feuille non tissée fibreuse, et où de préférence la feuille non tissée a une largeur dans la direction latérale (1) de l'article absorbant (1) qui est supérieure à une largeur du film polymérique dans la direction latérale (T) de l'article absorbant (1).

11. Article absorbant (1) selon l'une quelconque des revendications précédentes, où les portions d'ancrage élastique de jambe (30, 31) sont formées par un matériau non tissé fibreux.

12. Article absorbant (1) selon l'une quelconque des revendications précédentes, où un troisième rabat latéral (43) s'étend du premier bord latéral (6) de l'article absorbant (1) dans la direction latérale (1) de l'article absorbant (1) et un quatrième rabat latéral (44) s'étend du deuxième bord latéral (8) de l'article absorbant (1) dans la direction latérale de l'article (1), le troisième et quatrième rabats latéraux (43, 44) étant disposés au deuxième bord de taille (4).

13. Article absorbant (1) selon la revendication 12, où le troisième et quatrième rabats latéraux (43, 44) ont chacun une portion d'extrémité intérieure qui est fermement attachée entre la feuille supérieure (9) et l'agencement de feuille arrière (10) dans une troisième et quatrième zones d'ancrage de rabat, chaque zone d'ancrage de rabat ayant une extension latérale dans la direction latérale de l'article absorbant (1) entre un bord intérieur et un bord extérieur de la zone d'ancrage de rabat et ayant une extension longitudinale dans la direction longitudinale (L) de l'article absorbant (1) entre un bord de taille du rabat latéral (43, 44) et un bord de jambe correspondant du rabat latéral (43, 44), et de préférence où les portions d'ancrage élastique de jambe (30,31) sont complètement ou partiellement disposées latéralement à l'extérieur des bords intérieurs correspondants de la troisième et quatrième zones d'ancrage de rabat (43, 44).

14. Article absorbant (1) selon l'une quelconque des revendications 12 et 13, où le premier et deuxième éléments élastiques de jambe (18, 19) terminent à une distance du deuxième bord de taille (4), de sorte que des deuxièmes portions de jambe libre d'élastique (46, 47) sont disposées au deuxième bord de taille (4), et de façon facultative, où les deuxièmes portions de jambe libre d'élastique (46, 47) sont formées par le premier et deuxième éléments élastiques de jambe (18, 19) étant rétractés du deuxième bord de taille (4).

15. Article absorbant (1) selon l'une quelconque des revendications précédentes, où l'agencement de feuille arrière (10) est non extensible dans la direction latérale (T) de l'article absorbant (1).
